# EUROPÄISCHE PATENTSCHRIFT

(11) **EP 0 507 991 B1**
(45) Veröffentlichungstag und Bekanntmachung des Hinweises auf die Patenterteilung: **09.10.1996**
(21) Anmeldenummer: 91119828.1
(22) Anmeldetag: 21.11.1991
(51) Int. Cl.: G02B 6/42, A61B 17/36

(54) **Adapter für eine Lichtleitereinkopplung**
Adaptor for a fiberoptical coupling
Adaptateur pour un couplage à fibre optique

(30) Priorität: 12.04.1991 DE 4112081
(43) Veröffentlichungstag der Anmeldung: 14.10.1992
(73) Patentinhaber: Dornier Medizintechnik GmbH, 81663 München (DE)
(72) Erfinder: Taige, Patricia, W-8012 Ottobrunn (DE)
(74) Vertreter: Frick, Gerhard, Dipl.-Ing.

(56) Entgegenhaltungen:
- EP-A- 0 380 775
- US-A- 4 785 805

## Beschreibung

Die Erfindung betrifft einen Adapter für eine Lichtleitereinkopplung eines Lasergeräts, bestehend aus einem hülsenförmigen Paßteil (1) zur Aufnahme eines Lichtwellenleiters (7), wobei Funktionsmittel zur Betätigung elektrischer und/oder mechanischer Sicherungsvorrichtungen des Lasergeräts bzw. Funktionsmittel für elektrische und/oder mechanische Sicherungsvorrichtungen des Lasergeräts vorgesehen sind.

Aus der DE 39 02 807 C1 ist ein Adapter zum Anschluß eines Lichtleiters an ein medizinisches Lasergerät bekannt geworden, der ein hülsenförmiges Paßteil enthält, in welchem axial die optische Kupplung des Lichtleiters angeordnet ist. Weiterhin ist ein Funktionsmittel - hier: Mikroschalter - vorgesehen, mit dessen Hilfe Funktionen des Lasergerätes beim Einstecken des Adapters gesteuert werden. Bei der Herstellung und beim Betrieb des Adapters hat es sich aber als nachteilig herausgestellt, daß der Adapter aus vielen Einzelteilen besteht, die zudem noch mittels Passungen ineinander gefügt sind und daß der Adapter nicht zur Anpassung an handelsübliche genormte Lichtleiterstecker geeignet ist.

Es ist daher Aufgabe der Erfindung, den bekannten Adapter derart weiterzuentwickeln, daß die o.g. Nachteile vermieden werden und daß das Einstecken des kompletten Adapters ohne eingefügten Lichtleiter verhindert wird. Diese Aufgabe wird durch einen nach den kennzeichnenden Merkmalen des Patentanspruchs 1 ausgebildeten Adapter in überraschend einfacher Weise gelöst. Vorteilhafte Weiterbildungen der Erfindung sind den kennzeichnenden Teilen der Unteransprüche zu entnehmen.

Der besondere Vorteil des Adapters liegt darin, daß der Griff und das Paßteil nur dann zusammengefügt werden können, wenn zuvor der Lichtleiter ordnungsgemäß im Paßteil angebracht wurde. Damit wird das Einstecken des Adapters ohne Lichtleiter in die Lichtleitereinkopplung wirkungsvoll verhindert, so daß keine Laserstrahlung ohne montierten Lichtleiter austreten kann.

Ausführungsbeispiele gemäß der Erfindung sind in der Zeichnung dargestellt und werden im folgenden näher beschrieben. Es zeigen:
- Fig. 1a: einen zerlegten Adapter;
- Fig. 1b: einen Adapter von Fig. 1a während des Zusammenbaus;
- Fig. 2a: ein weiteres Beispiel des Adapters;
- Fig. 2b: den Adapter von Fig. 2a während des Zusammenbaus.

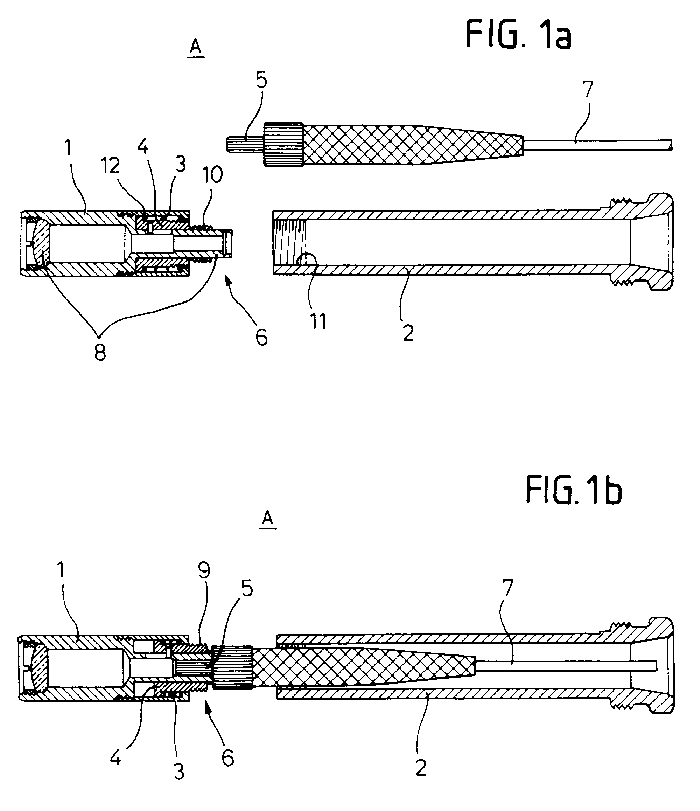

Die Fig. 1a zeigt die beiden Teile des Adapters A, das Paßteil 1 und den Griff 2. Das Paßteil 1 enthält die optische Kupplung 8 für den axial einzuführenden Lichtleiter 7 und das Anschlußgewinde 10 für das am proximalen Ende des Lichtleiters 7 befestigte Endteil 5 (hier: verschraubbarer Stecker).

Innerhalb des Paßteils 1 ist die axial gegen die Feder 3 verschiebbare Koppelhülse 4 gelagert, die ein zum Innengewinde 11 des Griffes 2 passendes Außengewinde 9 trägt. In der in Fig. 1a gezeigten Sperrstellung ist das Außengewinde 9 vollständig unter den am Paßteil 1 befestigten hülsenförmigen Mantel 12 zurückgezogen, so daß der Griff 2 nicht an das Paßteil 1 angeschraubt werden kann.

Erst wenn der Lichtleiter 7 mittels der Vorrichtung 5 mit dem Paßteil 1 verschraubt wird, gelangt die als Sperrmittel dienende Koppelhülse 4 von ihrer Sperr- in die Betriebslage (Fig. 1b), in der das Außengewinde 9 frei zugänglich ist. Damit kann der Adapter A mittels des über den Lichtleiter 7 geschobenen Griffes 2 vervollständigt werden. Wird der komplette Adapter A in die hier nicht dargestellte Lichtleitereinkopplung am Lasergerät eingeschoben, so werden dort Funktionsmittel betätigt, die so angeordnet oder geschaltet sind, daß nur mittels eines vollständigen Adapters A eine Laserstrahlung in Richtung auf den Lichtleiter 7 abgegeben wird. Da die Verbindung von Paßteil 1 und Griff 2 ohne eingefügten Lichtleiter 7 nicht möglich ist, kann nur noch das mißbräuchliche Einschieben vom Paßteil 1 oder Griff 2 allein in die Lichtleitereinkopplung versucht werden. Dies löst aber aufgrund der Anordnung der Funktionsmittel in der Lichtleitereinkopplung keine Abgabe von Laserstrahlung aus.

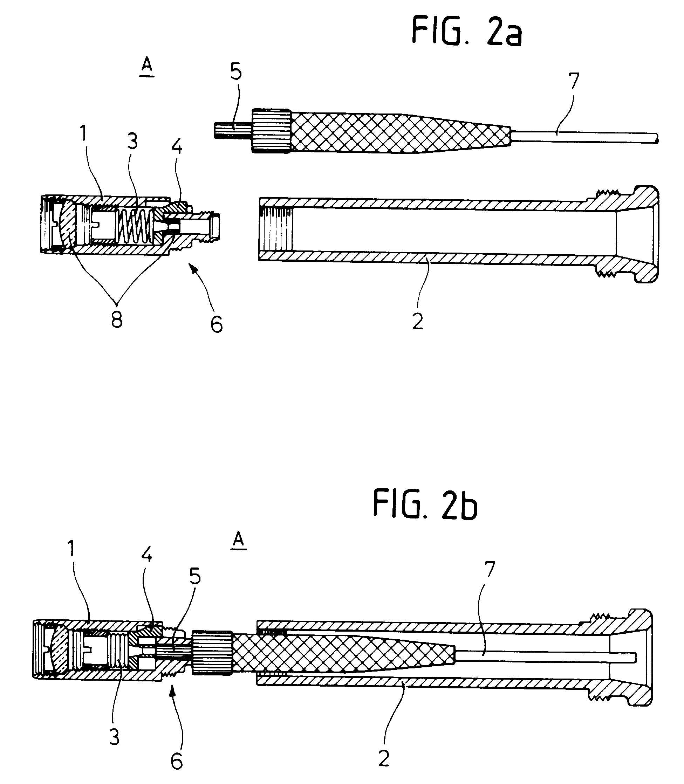

In der Fig. 2a sind die beiden Teile des Adapters A, das Paßteil 1 und der Griff 2 getrennt voneinander dargestellt. Das Paßteil 1 besteht im wesentlichen aus einer hülsenförmigen Fassung, in der die optische Kupplung 8 für den Lichtleiter 7 axial ausgerichtet gelagert ist. Im Bereich der Verbindungsstelle 6 zum rohrförmigen Griff 2 ist ein Außengewinde vorgesehen, das durch ein als winkelförmiger Sperrschieber ausgeführtes Sperrmittel 4 blockiert ist. Das Sperrmittel 4, das in der Fig. 2a in seiner Sperrstellung dargestellt ist, ist innerhalb des Paßteils 1 axial verschiebbar gelagert und wird in dieser Stellung mit Hilfe einer Druckfeder 3 gehalten.

Der Griff 2 besitzt an seinem zum Paßteil 1 weisenden Ende ein zum Außengewinde des Paßteils korrespondierendes Innengewinde. Wenn man nun versucht, den Griff 2 und das Paßteil 1 zusammenzuschrauben, ohne daß vorher der Lichtleiter 7 mit der darauf an dessen proximalen Ende angebrachten Vorrichtung (verschraubbarer Stecker) in das Paßteil 1 eingesteckt und befestigt wurde, so wird dieser Vorgang durch den Sperrschieber 4 blockiert. Der Sperrschieber kippt bei exzentrischer Krafteinleitung, die durch den gewindeseitigen Rand des Griffes 2 verursacht wird, ein wenig im Bereich seiner Sperrlage und verklemmt mit seinem angefasten Winkelarm in seiner Führungsnut im Paßteil 1. Das Einschieben des Griffes 2 allein würde nicht ein Einschalten der Laserstrahlung bewirken, da innerhalb der Lichtleitereinkopplung mindestens ein Tastschalter vorgesehen ist, der mit dem eingeschobenen Paßteil 1 korrespondiert und somit bei Fehlen eines der beiden Adapterteile 1, 2 die Abstrahlung verhindert. Zusätzlich kann im Paßteil 1 ein weiteres Sperrmittel, z.B. in Form eines über die Außenkontur hinausragenden Stiftes, vorgesehen sein, das das mißbräuchliche Einschieben des Paßteils 1 ohne den damit verbunden Griff in die Lichtleitereinkopplung verhindert.

Erst wenn der Adapter, wie in Fig. 2b gezeigt, ordnungsgemäß zusammengebaut und in die Lichtleitereinkopplung eingeschoben wurde, wird vom Lasergerät Strahlung abgegeben. Beim Zusammenbau wird zunächst die Vorrichtung 5 in das Paßteil 1 gesteckt und anschließend verschraubt, wodurch das proximale Ende des Lichtleiters 7 bezüglich der optischen Kupplung 8 lagefixiert ist. Gleichzeitig wird mit Hilfe der Vorrichtung 5 der Sperrschieber 4 gegen die Feder 3 axial verschoben bis das Gewinde an der Verbindungsstelle 6 freigegeben ist und der Griff 2 ordnungsgemäß aufgeschraubt werden kann.

## Patentansprüche

1. Adapter zum Anschluß eines Lichtwellenleiters an ein Lasergerät, bestehend aus einem hülsenförmigen Paßteil (1) zur Aufnahme des Lichtwellenleiters (7), wobei Funktionsmittel zur Betätigung elektrischer und/oder mechanischer Sicherungsvorrichtungen des Lasergeräts oder Funktionsmittel für elektrische und/oder mechanische Sicherungsvorrichtungen des Lasergeräts vorgesehen sind, **gekennzeichnet** durch folgende Merkmale:
a) der Adapter (A) besteht aus zwei lösbar miteinander verbundenen Teilen, dem Paßteil (1) und einem Griff(2), wobei das Paßteil (1) mit dem Lasergerät verbindbar ist;
b) im Bereich der Verbindungsstelle (6) zwischen dem Paßteil (1) und dem Griff (2) ist im Paßteil (1) eine axial bewegliche Koppelhülse (4) vorgesehen, die den mit einem Endteil (5) versehenen zu verbindenden Lichtleiter umgibt, wobei die Koppelhülse (4) gleichzeitig als Sperrmittel für die Verbindung des Paßteils (1) und des Griffs (2) ausgebildet ist und derart im Paßteil gelagert ist, daß mittels des Endteiles (5) des Lichtleiters (7), das axial am Sperrmittel angreift, das Sperrmittel (4) von seiner Sperrlage in seine Betriebslage überführt wird, wodurch die Verbindung zwischen Griff (2) und Paßteil (1) erst bei eingefügtem Endteil (5) des Lichtleiters (7) ermöglicht wird.

2. Adapter nach Anspruch 1, **dadurch gekennzeichnet,** daß das Sperrmittel gegen die Kraft einer Feder (3) verschiebbar und/oder verdrehbar angeordnet ist.

3. Adapter nach Anspruch 2 oder 3, **dadurch gekennzeichnet,** daß das Sperrmittel (4) als Schieber ausgeführt ist, der zur Überführung von der Sperr- in die Betriebslage mittels des Endteils (5) axial verschiebbar ist und der in seiner Sperrlage bei nichtaxialer Krafteinleitung bis zur Klemmung kippbar ist, wobei das Sperrmittel und der Griff (2) so ausgestaltet sind, daß die nicht axiale Krafteinleitung bei der Verbindung des Griffs (2) mit dem Paßteil (1) bei nicht montiertem Endteil (5) des Lichtwellenleiters auftritt.

4. Adapter nach einem der Ansprüche 1 bis 3, **dadurch gekennzeichnet,** daß das Paßteil (1) und der Griff(2) so ausgestaltet sind, daß beim Einschieben von Paßteil (1) und Griff (2) in die Lichtleitereinkopplung des Lasergeräts wenigstens ein Funktionsmittel betätigt wird, das die Abstrahlung von Laserleitung verhindert.

5. Adapter nach einem der Ansprüche 1 bis 4, **dadurch gekennzeichnet,** daß im Paßteil (1) ein weiteres Sperrmittel vorgesehen ist, welches ein Einschieben des Paßteils (1) in die Lichtleitereinkopplung verhindert, wenn der Griff(2) nicht mit dem Paßteil (1) verbunden ist.

## Claims

1. Adapter for connecting an optical waveguide to a laser instrument, comprising a sleeve-like fitting (1) for accommodating the optical waveguide (7), functional means being provided for actuating electrical and/or mechanical safety devices of the laser instrument or functional means being provided for electrical and/or mechanical safety devices of the laser instrument, characterized by the following features:
a) the adapter (A) consists of two parts, the fitting (1) and a handle (2), which are detachably connected to each other, the fitting (1) being able to be connected to the laser instrument;
b) in the region of the connecting point (6) between the fitting (1) and the handle (2), there is provided in the fitting (1) an axially movable coupling sleeve (4) that surrounds the optical fibre to be connected, which is provided with an end part (5), the coupling sleeve (4) being constructed at the same time as a blocking means for the connection of the fitting (1) and the handle (2) and being supported in the fitting in such a way that, by means of the end part (5) of the optical fibre (7), which end part engages axially on the blocking means, the blocking means (4) is transferred from its blocking position into its operating position, as a result of which the connection between the handle (2) and the fitting (1) is made possible only after the end part (5) of the optical fibre (7) has been inserted.

2. Adapter according to Claim 1, characterized in that the blocking means is arranged so as to be displaceable and/or rotatable counter to the force of a spring (3).

3. Adapter according to Claim 1 or 2, characterized in that the blocking means (4) is designed as a slide which can be displaced axially by means of the end part (5) to transfer it from the blocking position into the operating position and which, in its blocking position, can be tilted until it jams in the event of the non-axial introduction of force, the blocking means and the handle (2) being configured such that the non-axial introduction of force occurs if the handle (2) is connected to the fitting (1) without the end part (5) of the optical waveguide having been fitted.

4. Adapter according to one of Claims 1 to 3, characterized in that the fitting (1) and the handle (2) are configured such that, when the fitting (1) and handle (2) are pushed into the fibre-optic coupling of the laser instrument, at least one functional means is actuated which prevents the emission of laser power.

5. Adapter according to one of Claims 1 to 4, characterized in that there is provided in the fitting (1) a further blocking means which prevents the fitting (1) being pushed into the fibre-optic coupling if the handle (2) is not connected to the fitting (1).

## Revendications

1. Adaptateur pour le couplage d'une fibre optique à un appareil laser, comprenant un élément calibré (1) en forme de manchon qui reçoit la fibre optique (7), des moyens fonctionnels d'actionnement de dispositifs électriques et/ou mécaniques de sécurité de l'appareil laser ou des moyens fonctionnels pour des dispositifs électriques et/ou mécaniques de sécurité de l'appareil laser étant prévus, caractérisé par les caractéristiques suivantes:
a) l'adaptateur (A) est formé de deux parties qui sont liées l'une à l'autre de manière démontable, à savoir de l'élément calibré (1) et d'une poignée (2), l'élément calibré (1) pouvant être connecté à l'appareil laser;
b) il est prévu, dans la région du point d'assemblage (6) entre l'élément calibré (1) et la poignée (2), à l'intérieur de l'élément calibré (1), une douille de couplage (4) mobile axialement qui entoure la fibre optique à connecter pourvue d'un embout (5), la douille de couplage (4) étant agencée simultanément en moyen de verrouillage pour la liaison entre l'élément calibré (1) et la poignée (2) et étant montée dans l'élément calibré de telle sorte que le moyen de verrouillage (4) soit amené de sa position de verrouillage dans sa position de fonctionnement par l'embout (5) de la fibre optique qui agit axialement sur ledit moyen de verrouillage, la liaison entre le poignée (2) et l'élément calibré (1) n'étant possible que lorsque l'embout (5) de la fibre optique (7) est rentré.

2. Adaptateur selon la revendication 1, caractérisé par le fait que le moyen de verrouillage est disposé de manière à pouvoir coulisser et/ou tourner à l'encontre de la force d'un ressort (3).

3. Adaptateur selon la revendication 1 ou 2, caractérisé par le fait que le moyen de verrouillage (4) est réalisé sous la forme d'un élément coulissant qui coulisse axialement aux fins de passer de la position de verrouillage à la position de fonctionnement sous l'action de l'embout (5) et qui, dans sa position de verrouillage, bascule dans une position blocante lorsque la force n'agit pas axialement, le moyen de verrouillage et la poignée (2) étant agencés de telle sorte que la force non axiale apparaisse en lorsqu'on assemble la poignée (2) et l'élément calibré (1) alors que l'embout (5) de la fibre optique n'est pas monté.

4. Adaptateur selon l'une des revendications 1 à 3, caractérisé par le fait que l'élément calibré (1) et la poignée (2) sont agencés de manière telle que, lors de l'introduction de l'élément calibré (1) et de la poignée (2) dans le coupleur de fibre optique de l'appareil laser, au moins un moyen fonctionnel empêchant l'émission du rayonnement laser soit actionné.

5. Adaptateur selon l'une des revendications 1 à 4, caractérisé par le fait qu'il est prévu dans l'élément calibré (1) un moyen de verrouillage supplémentaire qui empêche l'introduction de l'élément calibré (1) dans le coupleur de fibre optique lorsque la poignée (2) n'est pas liée à l'élément calibré (1).
